# EUROPÄISCHE PATENTSCHRIFT

(11) **EP 0 531 361 B1**
(45) Veröffentlichungstag und Bekanntmachung des Hinweises auf die Patenterteilung: **25.10.1995**
(21) Anmeldenummer: 91909845.9
(22) Anmeldetag: 21.05.1991
(51) Int. Cl.: C07C 309/17, C07C 303/32

(54) **VERFAHREN ZUR HERSTELLUNG VON HELLFARBIGEN alpha-SULFOFETTSÄUREALKYLESTER-ALKALIMETALLSALZPASTEN**
PROCESS FOR PRODUCING LIGHT-COLOURED PASTES OF alpha-SULPHO FATTY ACID ALKYL ESTER ALKALI METAL SALTS
PROCEDE DE PRODUCTION DE PATES DE COULEUR CLAIRE EN SELS DE METAUX ALCALINS-ALKYLESTERS D'ACIDES GRAS alpha-SULFONIQUES

(30) Priorität: 30.05.1990 DE 4017467
(43) Veröffentlichungstag der Anmeldung: 17.03.1993
(73) Patentinhaber: Henkel Kommanditgesellschaft auf Aktien, 40191 Düsseldorf (DE)
(72) Erfinder: COLIGNON, Dietmar, D-4006 Erkrath (DE); DORRA, Erich, D-4000 Düsseldorf 13 (DE); PANTHEL, Günter, D-5657 Haan (DE); SCHMIDT, Wolfgang, D-4019 Monheim 2 (DE); WREDE, Norbert, D-4000 Düsseldorf 13 (DE)
(86) Internationale Anmeldenummer: EP9100951
(87) Internationale Veröffentlichungsnummer: WO9118874

(56) Entgegenhaltungen:
- EP-A- 0 222 237
- DE-B- 1 246 718
- DE-B- 1 248 645

## Beschreibung

Die Erfindung betrifft ein Verfahren zur Herstellung von hellfarbigen α-Sulfofettsäurealkylester-Alkalimetallsalzpasten, bei dem man das rohe Sulfonierungsprodukt unmittelbar nach der Sulfierreaktion in einer annähernd ideal durchmischten flüssigen Phase einer temperaturkontrollierten Nachreaktion über läßt bis ein Sulfiergrad von mindestens 90 % erreicht ist.

α-Sulfofettsäurealkylester-Alkalimetallsalze haben derzeit steigende Bedeutung als oberflächenaktive Substanzen für Wasch- und Reinigungsmittel aus nachwachsenden natürlichen Rohstoffen. Man erhält die α-Sulfofettsäurealkylester-Alkalimetallsalze nach bekannten Verfahren in Form von wässrigen Lösungen oder Pasten durch Neutralisation von α-Sulfofettsäurealkylestern, welch letztere durch Umsetzung von Fettsäureniedrigalkylestern mit gasförmigem SO₃ synthetisiert werden können. Als Basis für die Herstellung der α-Sulfofettsäurealkylester-Alkalimetallsalze dienen letzten Endes Fette und Öle natürlichen Ursprungs, aus denen die Fettsäureniedrigalkylester durch Fettspaltung und nachfolgende Veresterung der freien Fettsäuren mit niederen Alkanolen oder durch Umesterung der natürlichen Triglyceride mit niederen Alkanolen erhalten werden. Bei beiden Reaktionen ist Methanol als niederes Alkanol bevorzugt. Die Fettsäureniedrigalkylester stellen Gemische dar, in denen Fettsäurereste mit 6 bis 22 Kohlenstoffatomen vorkommen, wobei die Kettenlängenverteilung vom Ursprung der natürlichen Fette oder Öle abhängig ist. Diese Fettsäureestergemische werden häufig nicht als solche, sondern in Form bestimmter Fraktionen zur Synthese eingesetzt. Durch Sulfonierung der Fettsäureestergemische mit gasförmigem SO₃ erhält man saure α-Sulfofettsäurealkylester, die durch Neutralisation auf einen pH-Wert von 6 bis 8 in wässrige Pasten von α-Sulfofettsäurealkyleseter-Alkalimetallsalzen überführt werden. Die rohen α-Sulfofettsäurealkylester und gegebenenfalls auch ihre Alkalimetallsalze stellen mehr oder weniger gefärbte Produkte dar, die in der Regel vor und/oder nach der Neutralisation einer Behandlung mit üblichen Bleichmitteln unterworfen werden müssen.

Die Sulfonierung der Fettsäurealkylester erfolgt üblicherweise mit gasförmigem SO₃ bei Temperaturen von 30 bis 100 °C, wobei das Molverhältnis von Fettsäureester : SO₃ im Bereich von 1 : 1,2 bis 1 : 1,8 liegt. Die Reaktion der Fettsäurealkylester mit SO₃ vollzieht sich in zwei Teilschritten. In einem ersten, schnellen Teilschritt reagieren zwei SO₃-Moleküle mit einem Molekül Fettsäurealkylester zu einem gemischten Anhydrid aus α-Sulfofettsäure und Alkylschwefelsäure. In einem langsamen, zweiten Reaktionsschritt wirkt das gemischte Anhydrid als Sulfiermittel für noch nicht umgesetzten Fettsäurealkylester. Der erste Reaktionsschritt läuft in dem jeweils verwendeten üblichen Sulfierreaktor ab, beispielsweise in einem Fallfilmreaktor oder in einer Sulfierkaskade. Über die Durchführung des zweiten Reaktionsschrittes findet sich bei den bekannten Verfahren nur spärliche Angaben, die sich im allgemeinen auf die Reaktionstemperatur und allenfalls die Reaktionszeit beschränken. So enthält die DE-OS 31 23 681 die Angabe, daß das Sulfonierungsprodukt bei 30 bis 100 °C gealtert werden soll. Nach der DE-OS 33 34 517 wird das Sulfonierungsprodukt 10 bis 20 Minuten bei 80 bis 100 °C gealtert.

Bei den Arbeiten, die zu dieser Erfindung geführt haben, wurde festgestellt, daß die Reaktionsführung im zweiten Schritt der Sulfonierungsreaktion von entscheidender Bedeutung für den Sulfiergrad und die Qualität des Endproduktes ist. Es wurde festgestellt, daß die Produktqualität in hohem Maße von einer exakten Temperaturführung und einer möglichst engen Verweilzeitverteilung bei der Nachreaktion abhängig ist. Kurze Verweilzeiten führen zu α-Sulfofettsäurealkylester-Alkalimetallsalzpasten mit sehr guter Bleichbarkeit aber mit unzureichendem Sulfiergrad. Lange Verweilzeiten führen zu hohen Sulfiergraden aber auch zu stark gefärbten Produkten, die nach der Neutralisation nicht auf die erforderlichen Farbwerte gebleicht werden können.

Übliche Verweilzeitinstallationen können die Forderung nach enger Verweilzeitverteilung bei gleichzeitiger exakter Temperaturführung nicht erfüllen. Gebräuchliche Reaktoren, die lediglich mit einem Heiz- und Kühlkreislauf betrieben werden, führen wegen der ständigen Rückvermischung zu einem viel zu breiten Verweilzeitspektrum. Nachgeschaltete temperierte Rohrschlangen müssen stets mit einer voll ausgebildeten turbulenten Strömung, d.h. unter Plug-Flow-Bedingungen betrieben werden und können deshalb nicht unabhängig von der Durchsatzleistung des Sulfierreaktors betrieben werden.

Die Erfindung geht deshalb auf die Aufgabenstellung zurück, ein Verfahren aufzufinden, das es gestattet, bei der Herstellung von hellfarbigen α-Sulfofettsäurealkylester-Alkalimetallsalzen durch Umsetzung von Fettsäurealkylestern mit gasförmigem SO₃ und Neutralisation mit wässrigen Alkalimetallhydroxidlösungen das rohe Sulfonierprodukt unter exakter Temperaturführung und gleichzeitig vorhandener enger Verweilzeitverteilung einer Nachreaktion zu überlassen, bevor es in an sich bekannter Weise neutralisiert und gebleicht wird.

Es konnte gezeigt werden, daß eine dem Sulfierreaktor nachgeschaltete Rührkesselkaskade üblicher Bauart mit Heiz- und Kühlvorrichtungen die Bedingungen, die der zweite Reaktionsschritt der Fettsäurealkylestersulfonierung hinsichtlich Temperaturführung und Verweilzeitverhalten erfordert, ideal erfüllt. Die Kaskade kann dabei aus beliebig vielen, vorzugsweise jedoch vier hintereinander geschalteten Rührkesseln bestehen. Wenn die Rührkesselkaskade aus vier und mehr Einheiten besteht, gleicht sich ihr Verweilzeitverhalten dem eines turbulent durchströmten Rohres an. Die mittlere Verweilzeit für den zweiten Sulfonierungsschritt kann dabei leicht über das Flüssigkeitsniveau in den einzelnen Rührkesseln eingestellt werden. Mit Hilfe mehrerer hintereinander geschalteter, ideal durchmischter Rührkessel kann sowohl ein isothermer Reaktionsverlauf als auch jedes gewünschte andere Temperaturprofil realisiert werden.

Gegenstand der Erfindung ist ein Verfahren zur Herstellung von hellfarbigen α-Sulfofettsäurealkylester-Alkalimetallsalzpasten durch Umsetzung von Fettsäurealkylestern mit gasförmigem SO₃, anschließende Nachreaktion in angenähert ideal durchmischter flüssiger Phase und Neutralisation mit wässrigen Alkalimetallhydroxidlösungen, bei dem man
a) Fettsäurealkylester in einem üblichen Sulfierreaktor mit einem mindestens 10 %igen molaren Überschuß an SO₃ zur Umsetzung bringt,
b) das rohe Sulfonierungsprodukt einer mindestens zwei Stufen umfassenden Rührkesselkaskade mit Heiz- und Kühlvorrichtungen zuführt und sie dort unter mechanischer Bewegung einer temperaturkontrollierten Nachreaktion bis zum Erreichen eines Sulfiergrades von mindestens 90 % überläßt und
c) das auf diese Weise gealterte Sulfonierungsprodukt in an sich bekannter Weise zu α-Sulfofettsäurealkylester-Alkalimetallsalzpasten weiter verarbeitet.

Die Sulfierung der Fettsäureester erfolgt mit gasförmigem SO₃ als Sulfonierungsreagenz bei Temperaturen von 30 bis 100 °C. Dabei wird das SO₃ mit Luft oder Stickstoff verdünnt, vorzugsweise in Form eines Gasgemisches mit 1 bis 10 Vol.-% SO₃ mit dem Fettsäurestern in Berührung gebracht. Die Menge des SO₃ wird vorzugsweise so bemessen, daß das Molverhältnis von Fettsäureester zu SO₃ im Bereich von 1 : 1,2 bis 1 : 1,8 liegt. Diese Umsetzung kann in üblichen, für die Sulfonierung von organischen Verbindungen wie Fettalkoholen, Alkylbenzolen oder Olefinen geeigneten Reaktoren, insbesondere in Fallfilmreaktoren oder mehrstufigen Rührkesselkaskaden durchgeführt werden.

Die temperaturkontrollierte Nachreaktion wird in einer Rührkesselkaskade mit Heiz- und Kühlvorrichtungen bekannter Bauart durchgeführt. Die Rührkesselkaskade kann 2 bis 6 Stufen umfassen, wobei Vorrichtungen mit 4 Stufen bevorzugt sind. Die Heiz- und Kühlvorrichtungen der Rührkesselkaskade bestehen vorzugsweise aus Rohrschlangen oder Wärmeübertragungsmänteln.

Das rohe Sulfonierungsprodukt wird zunächst in die Stufe der Rührkesselkaskade eingebracht, in der es über einen Zeitraum von 1 bis 60, vorzugsweise 20-40 min. bei einer Temperatur von 60 bis 100 °C gerührt wird, ehe es anschließend in mindestens eine weitere Stufe der Rührkesselkaskade gelangt in der es wiederum über einen Zeitraum von 1 bis 60, vorzugsweise 20-40 min. bei einer Temperatur von 80 bis 90 °C intensiv gerührt wird, bis ein Sulfiergrad von 90, vorzugsweise 94-98 % erreicht ist.

Bei der temperaturkontollierten Nachreaktion soll das Sulfonierungsprodukt in angenähert ideal durchmischter flüssiger Phase vorliegen. Die hierfür erforderliche mechanische Bewegung des Sulfonierungsproduktes wird in der Rührkesselkaskade durch Rühren, Zufuhr des Produktes unter Druck oder durch eingebaute Umlenkschikanen bewirkt.

Bei der erfindungsgemäßen Herstellung von hellfarbigen α-Sulfofettsäurealkylester-Alkalimetallsalzpasten wird das gealterte Sulfonierungsprodukt im Anschluß an die temperaturkontrollierte Nachreaktion in an sich bekannter Weise neutralisiert und gebleicht.

Die Neutralisation des gealterten Sulfonierungsproduktes erfolgt mit wässrigen Alkalimetallhydroxidlösungen, vorzugsweise mit wässrigen Natriumhydroxidlösungen.

Die rohen Sulfonierungsprodukte und gegebenenfalls auch ihre Alkalimetallsalze sind mehr oder weniger gefärbte Substanzen. Aus diesem Grund muß vor und/oder nach ihrer Neutralisation eine Bleichung nach bekannten Verfahren durchgeführt werden, wobei als Bleichmittel vorzugsweise wässrige Wasserstoffperoxid- und/oder Hypochloritlösungen eingesetzt werden. Eine Bleichung vor der Neutralisation mit Wasserstoffperoxid ist in der DE-PS 11 79 931 beschrieben. Nach der DE-AS 12 34 709 wird in einer ersten Bleichstufe zunächst der saure α-Sulfofettsäurealkylester mit wässriger Wasserstoffperoxidlösung behandelt. Anschließend wird das teilgebleichte Sulfonierungsprodukt neutralisiert, bevor es in einer zweiten Bleichstufe der Einwirkung von weiterer Wasserstoffperoxidlösung oder von wässriger Hypochloritlösung ausgesetzt wird. Nach der DE-OS 33 19 591 wird das teilneutralisierte Sulfonierungsprodukt zunächst bei pH-Werten zwischen 7 und 11 mit wässriger Hypochloritlösung gebleicht. Im Anschluß daran wird bei pH-Werten von ≦ 7 wässrige Wasserstoffperoxidlösung zugesetzt, um die erreichten Farbwerte zu stabilisieren.

Unter Fettsäurealkylestern im Sinne der Erfindung werden Niedrigalkylester von gesättigten Fettsäuren verstanden, insbesondere Ester von Fettsäuren mit 10 bis 18 Kohlenstoffatomen und gesättigten aliphatischen Alkoholen mit 1 bis 4 Kohlenstoffatomen. Grundsätzlich kann man von einzelnen Fettsäurealkylestern ausgehen. In der Regel verwendet man als Ausgangsmaterial jedoch Estergemische, wie sie aus Fetten und Ölen natürlichen Ursprungs entweder durch Esterspaltung und nachfolgende Veresterung mit niederen Alkanolen oder durch Umesterung mit niederen Alkanolen nach bekannten Verfahren erhältlich sind, wobei die entsprechenden Fettsäuremetylestergemische wiederum bevorzugt sind. Soweit die auf diese Weise erhaltenen Fettsäureestergemische größere Anteile an Estern von Fettsäuren mit weniger als 10 Kohlenstoffatomen enthalten, werden diese "Vorlauffettsäureester" in der Regel durch Destillation abgetrennt. Die Fettsäureester dürfen außer der CH₂-Gruppe in α-Stellung zur Estergruppierung keine sulfatierbaren oder sulfonierbaren Gruppen enthalten. Aus diesem Grund kommen Hydroxyfettsäureester oder Hydroxyfettsäureester enthaltende Gemische nicht als Ausgangsmaterial in Betracht. Fettsäureestergemische, die nicht zu vernachlässigende Mengen an Estern ungesättigter Fettsäuren enthalten, insbesondere solche Ester, die eine Jodzahl über 5 aufweisen, sind erst nach einer Absättigung der Doppelbindungen im Zuge einer hydrierenden Härtung nach bekannten Verfahren als Ausgangsmaterial geeignet. Bei der hydrierenden Härtung werden die Jodzahlwerte der Estergemische vorzugsweise auf Werte von 0,2 und kleiner vermindert.

### Beispiele

### Beispiel 1 bis 6

Als Ausgangsmaterial wurde ein technischer Palmitin-/Stearinsäuremethylester (in Gew.-% nach der Kettenlänge im Fettsäurerest: 0,2 C₁₂; 1,2 C₁₄; 61,4 C₁₆; 0,9 C₁₇; 35,9 C₁₈; 0,4 C₂₀; mittleres Molgewicht 281,5; Säurezahl 1,1; Jodzahl 0,1; Verseifungszahl 202,1) verwendet. Der Fettsäuremethylester wurde kontinuierlich in einem üblichen Fallfilmreaktor bei 80 °C mit einem SO₃-Luftgemisch (5 Vol.-% SO₃) im Molverhältnis 1 : 1,25 sulfoniert. Einzelne Chargen des resultierenden Reaktionsgemisches wurden in einer aus vier Rührkesseln bestehenden Verweilzeitkaskade mit Verweilzeiten von 10, 30 und 60 Minuten bei 80 und 90 °C der Nachreaktion unterworfen.

Im Anschluß an die Nachreaktion wurden die gealterten Chargen mit wässriger Natriumhydroxidlösung neutralisiert. Mit der Neutralisationsbase wurde wässrige Wasserstoffperoxidlösung in das Reaktionsgemisch eingearbeitet, wobei Wasserstoffperoxid in einer Menge von 1,5 Gew.-%, berechnet als 100 %ige Substanz und bezogen auf WAS, zugesetzt wurde. Im Anschluß an die Neutralisation wurden alle Chargen 20 Stunden lang bei einer Temperatur von 80 °C gerührt. Danach wurden die Klett-Farbzahlen wurden an 5 Gew.-% Waschaktivsubstanz enthaltenden Lösungen bei pH 7,5 in einer 5 cm-Küvette unter Verwendung eines Blaufilters (420 nm) gemessen.

In der nachfolgenden Tabelle sind für jedes Beispiel die Nachreaktionstemperatur und die Verweilzeit sowie der erzielte Sulfoniergrad und die dazugehörende Klett-Farbzahl angegeben.

**Tabelle**

| Sulfiergrad und Klett-Farbzahl in Abhängigkeit von Nachreaktionstemperatur und Verweilzeit | | | | |
|---|---|---|---|---|
| Beispiel | Temperatur (°C) | Verweilzeit (min) | Sulfiergrad (%) | Klett-Farb-Zahl |
| 1 | 80 | 10 | 90 | 100 |
| 2 | 80 | 30 | 94 | 200 |
| 3 | 80 | 60 | 97 | 250 |
| 4 | 90 | 10 | 92 | 150 |
| 5 | 90 | 30 | 96 | 200 |
| 6 | 90 | 60 | 97 | 400 |

## Patentansprüche

1. Verfahren zur Herstellung von hellfarbigem α-Sulfofettsäurealkylester-Alkalimetallsalzpasten durch Umsetzung von Fettsäurealkylestern mit gasförmigem SO₃, anschließende Nachreaktion in angenähert ideal durchmischter flüssiger Phase und Neutralisation mit wässrigen Alkalimetallhydroxidlösungen, dadurch gekennzeichnet, daß man
a) Fettsäurealkylester in einem üblichen Sulfierreaktor mit einem mindestens 10 %igen molaren Überschuß an SO₃ zur Umsetzung bringt,
b) das rohe Sulfonierungsprodukt einer mindestens zwei Stufen umfassenden Rührkesselkaskade mit Heiz- und Kühlvorrichtungen zuführt und sie dort unter mechanischer Bewegung einer temperaturkontollierten Nachreaktion bis zum Erreichen eines Sulfiergrades von mindestens 90 % über läßt und
c) das auf diese Weise gealterte Sulfonierungsprodukt in an sich bekannter Weise zu α-Sulfofettsäurealkylester-Alkalimetallsalzpasten weiter verarbeitet.

2. Verfahren nach Anspruch 1, dadurch gekennzeichnet, daß man gasförmiges SO₃ in Form eines SO₃/Luft- oder SO₃/Stickstoffgemisches mit 1 bis 10 Vol.-% SO₃ einsetzt.

3. Verfahren nach mindestens einem der Ansprüche 1 und 2, dadurch gekennzeichnet, daß man die Umsetzung mit SO₃ in einem Molverhältnis Fettsäurealkylester : SO₃ im Bereich von 1 : 1,2 bis 1 : 1,8 durchführt.

4. Verfahren nach mindestens einem der Ansprüche 1 bis 3, dadurch gekennzeichnet, daß man die Umsetzung mit SO₃ in einem Fallfilmreaktor oder in einer mehrstufigen Sulfierkaskade durchführt.

5. Verfahren nach mindestens einem der Ansprüche 1 bis 4, dadurch gekennzeichnet, daß die Rührkesselkaskade 3 bis 6 Stufen, vorzugsweise 4 Stufen, umfaßt.

6. Verfahren nach mindestens einem der Ansprüche 1 bis 5, dadurch gekennzeichnet, daß die Heiz- und Kühlvorrichtungen der Rührkesselkaskade aus Rohrschlangen oder Wärmeübertragungsmänteln bestehen.

7. Verfahren nach mindestens einem der Ansprüche 1 bis 6, dadurch gekennzeichnet, daß man das exotherm reagierende Reaktionsgemisch in allen Stufen der Rührkesselkaskade auf Temperaturen im Bereich von 60 bis 100 °C, vorzugsweise auf Temperaturen im Bereich von 80 bis 90 °C, hält.

8. Verfahren nach mindestens einem der Ansprüche 1 bis 7, dadurch gekennzeichnet, daß man die mechanische Bewegung des rohen Sulfonierungsproduktes in der Rührkesselkaskade durch Rühren, Zufuhr der Mischung unter Druck oder eingebaute Umlenkschikanen bewirkt.

9. Verfahren nach mindestens einem der Ansprüche 1 bis 8, dadurch gekennzeichnet, daß man das rohe Sulfonierungsprodukt so lange in der Rührkesselkaskade beläßt, bis ein Sulfiergrad von 94 bis 98 % erreicht ist.

10. Verfahren nach mindestens einem der Ansprüche 1 bis 9, dadurch gekennzeichnet, daß man das rohe Sulfonierungsprodukt 10 bis 60 Minuten, vorzugsweise 20 bis 40 Minuten, in der Rührkesselkaskade beläßt.

11. Verfahren nach mindestens einem der Ansprüche 1 bis 10, dadurch gekennzeichnet, daß man das gealterte Sulfonierungsprodukt im Anschluß an die temperaturkontrollierte Nachreaktion neutralisiert und bleicht.

12. Verfahren nach mindestens einem der Ansprüche 1 bis 11, dadurch gekennzeichnet, daß man das gealterte Sulfonierungsprodukt mit wässriger Alkalimetallhydroxidlösung, vorzugsweise mit wässriger Natriumhydroxidlösung, neutralisiert.

13. Verfahren nach mindestens einem der Ansprüche 1 bis 12, dadurch gekennzeichnet, daß man das Sulfonierungsprodukt vor und/oder nach der Neutralisation mit Wasserstoffperoxid und/oder Hypochlorit bleicht.

14. Verfahren nach mindestens einem der Ansprüche 1 bis 13, dadurch gekennzeichnet, daß man als Ausgangsmaterial Fettsäuremethylester einsetzt.

15. Verfahren nach mindestens einem der Ansprüche 1 bis 14, dadurch gekennzeichnet, daß man als Ausgangsmaterial durch Umesterung von natürlichen Fetten und/oder Ölen mit Methanol erhältliche Fettsäuremethylestergemische einsetzt.

## Claims

1. A process for the production of light-colored α-sulfofatty acid alkyl ester alkali metal salt pastes by reaction of fatty acid alkyl esters with gaseous SO₃, subsequent after-reaction in a substantially ideally mixed liquid phase and neutralization with aqueous alkali metal hydroxide solutions, characterized in that
a) fatty acid alkyl esters are reacted with an at least 10% molar excess of SO₃ in a typical sulfonation reactor,
b) the crude sulfonation product is fed to an at least two-stage cascade of stirred tanks with heating and cooling systems in which it is subjected with mechanical agitation to a temperature-controlled after-reaction until a degree of sulfonation of at least 90% is reached and
c) the sulfonation product aged in this way is further processed in known manner to α-sulfofatty acid alkyl ester alkali metal salt pastes.

2. A process as claimed in claim 1, characterized in that gaseous SO₃ is used in the form of an SO₃/air of SO₃/nitrogen mixture containing 1 to 10% by volume SO₃.

3. A process as claimed in at least one of claims 1 and 2, characterized in that the reaction with SO₃ is carried out in a molar ratio of fatty acid alkyl ester to SO₃ of 1 : 1.2 to 1 : 1.8.

4. A process as claimed in at least one of claims 1 to 3, characterized in that the reaction with SO₃ is carried out in a falling-film reactor or in a multistage sulfonation cascade.

5. A process as claimed in at least one of claims 1 to 4, characterized in that the cascade of stirred tanks comprises 3 to 6 stages and preferably 4 stages.

6. A process as claimed in at least one of claims 1 to 5, characterized in that the heating and cooling systems of the cascade of stirred tanks consist of pipe coils or heat-transfer jackets.

7. A process as claimed in at least one of claims 1 to 6, characterized in that, in all the stirred tanks of the cascade, the exothermically reacting reaction mixture is kept at temperatures in the range from 60 to 100°C and preferably at temperatures in the range from 80 to 90°C.

8. A process as claimed in at least one of claims 1 to 7, characterized in that the mechanical agitation of the crude sulfonation product in the cascade of stirred tanks is obtained by stirring, by introduction of the mixture under pressure or by built-in chicane-like baffles.

9. A process as claimed in at least one of claims 1 to 8, characterized in that the crude sulfonation product is left in the cascade of stirred tanks until a degree of sulfonation of 94 to 98% is reached.

10. A process as claimed in at least one of claims 1 to 9, characterized in that the crude sulfonation product is left in the cascade of stirred tanks for 10 to 60 minutes and preferably for 20 to 40 minutes.

11. A process as claimed in at least one of claims 1 to 10, characterized in that the aged sulfonation product is neutralized and bleached after the temperature-controlled after-reaction.

12. A process as claimed in at least one of claims 1 to 11, characterized in that the aged sulfonation product is neutralized with aqueous alkali metal hydroxide solution, preferably with aqueous sodium hydroxide solution.

13. A process as claimed in at least one of claims 1 to 12, characterized in that the sulfonation product is bleached with hydrogen peroxide and/or hypochlorite before and/or after neutralization.

14. A process as claimed in at least one of claims 1 to 13, characterized in that fatty acid methyl ester is used as the starting material.

15. A process as claimed in at least one of claims 1 to 14, characterized in that fatty acid methyl ester mixtures obtainable by transesterification of natural fats and/or oils with methanol are used as the starting material.

## Revendications

1. Procédé de préparation de pâtes d'esters alkyliques d'α-sulfoacides gras-sel de métal alcalin par réaction d'esters alkyliques d'acide gras avec du SO₃ gazeux puis post-réaction consécutive en phase liquide presque idéalement mélangée et neutralisation avec des solutions aqueuses d'hydroxyde de métal alcalin, caractérisé en ce que;
a) on fait réagir de l'ester alkylique d'acide gras dans un réacteur usuel de sulfonation avec au moins un excès 10 % molaire de SO₃,
b) on soumet le produit brut de sulfonation d'une cascade de réacteurs à agitation comprenant au moins deux étapes, équipées de dispositifs de chauffage et de refroidissement, et on l'y soumet sous agitation mécanique à une post-réaction à température contrôlée jusqu'à l'obtention d'un degré de sulfonation d'au moins 90 % et,
c) on transforme le produit de sulfonation muni de cette façon d'une manière déjà connue en pâtes d'ester alkylique d'α-sulfoacide gras-sel de métal alcalin.

2. Procédé selon la revendication 1, caractérisé en ce qu'on utilise du SO₃ gazeux sous forme d'un mélange SO₃/air ou SO₃/azote de 1 à 10 % en volume de SO₃.

3. Procédé selon au moins l'une des revendications 1 à 2, caractérisé en ce qu'on réalise la réaction avec SO₃ en une proportion molaire ester alkyliques d'acides gras : SO₃ dans l'intervalle de 1 : 1,2 à 1 : 1,8.

4. Procédé selon au moins l'une des revendications 1 à 3, caractérisé en ce qu'on réalise la réaction avec SO₃ dans un réacteur à ruissellement ou dans une cascade de sulfonation à plusieurs étapes.

5. Procédé selon au moins l'une des revendications 1 à 4, caractérisé en ce que la cascade de réacteurs à agitation comprend 3 à 6 étapes, de préférence 4 étapes.

6. Procédé selon au moins l'une des revendications 1 à 5, caractérisé en ce que les dispositifs de chauffage et de refroidissement de la cascade de réacteurs à agitation sont constitués de serpentins ou de doubles enveloppes d'échange thermique.

7. Procédé selon au moins l'une des revendications 1 à 6, caractérisé en ce qu'on maintient le mélange réactif exothermique à toutes les étapes de la cascade de réacteurs à agitation à des températures comprises dans le domaine de 60 à 100°C, de préférence à des températures comprises dans le domaine de 80 à 90°C.

8. Procédé selon au moins l'une des revendications 1 à 7, caractérisé en ce qu'on réalise le mouvement mécanique du produit brut de sulfonation dans la cascade de réacteurs à agitation par introduction du mélange sous pression ou par des chicanes de déviation.

9. Procédé selon au moins une des revendications 1 à 8, caractérisé en ce qu'on laisse le produit brut de sulfonation dans la cascade de réacteurs à agitation jusqu'à ce qu'on obtienne un degré de sulfonation de 94 à 98 %.

10. Procédé selon au moins l'une des revendications 1 à 9, caractérisé en ce qu'on laisse le produit brut de sulfonation 10 à 60 minutes, de préférence 20 à 40 minutes dans la cascade de réacteurs à agitation.

11. Procédé selon au moins l'une des revendications 1 à 10, caractérisé en ce qu'on neutralise et blanchit le produit de sulfonation vieilli après la post-réaction à température contrôlée.

12. Procédé selon au moins l'une des revendications 1 à 11, caractérisé en ce qu'on neutralise le produit de sulfonation vieilli avec de la solution aqueuse d'hydroxyde de métal alcalin, de préférence avec de la solution aqueuse d'hydroxyde de sodium.

13. Procédé selon au moins l'une des revendications 1 à 12, caractérisé en ce qu'on blanchit le produit de sulfonation avant et/ou après la neutralisation avec un peroxyde d'hydrogène et/ou de l'hypochlorite.

14. Procédé selon au moins l'une des revendications 1 à 13, caractérisé en ce qu'on utilise l'ester méthylique d'acide gras comme matériau de départ.

15. Procédé selon au moins l'une des revendications 1 à 14, caractérisé en ce qu'on utilise comme matériau de départ des mélanges d'esters méthyliques d'acides gras qu'on peut obtenir par transestérification d'huiles et/ou de graisses naturelles par le méthanol.
